# EUROPEAN PATENT SPECIFICATION

(11) **EP 4 075 239 B1**
(45) Date of publication and mention of the grant of the patent: **03.06.2026**
(21) Application number: 21169009.4
(22) Date of filing: 16.04.2021
(51) Int. Cl.: G06F 3/01, G06V 20/20, G06Q 10/00, G16H 40/63, G06F 16/583, G06F 16/587, G06Q 10/20, G06V 10/94, G09B 5/02

(54) **METHOD OF OPERATION OF A COMPUTER ASSISTED REALITY SYSTEM FOR A MEDICAL APPARATUS, COMPUTER ASSISTED REALITY SYSTEM AND COMPUTER PROGRAM PRODUCT**
VERFAHREN ZUM BETRIEB EINES COMPUTERGESTÜTZTEN REALITÄTSSYSTEMS FÜR EIN MEDIZINISCHES GERÄT, COMPUTERGESTÜTZTES REALITÄTSSYSTEM UND COMPUTERPROGRAMMPRODUKT
PROCÉDÉ DE FONCTIONNEMENT D'UN SYSTÈME DE RÉALITÉ ASSISTÉ PAR ORDINATEUR POUR UN APPAREIL MÉDICAL, SYSTÈME DE RÉALITÉ ASSISTÉ PAR ORDINATEUR ET PRODUIT PROGRAMME INFORMATIQUE

(43) Date of publication of application: 19.10.2022
(73) Proprietor: Baxter Medical Systems GmbH + Co. KG, 07318 Saalfeld (DE)
(72) Inventor: YAP, Cheah Chang, Batesville, 47006 (US); SCHULZE, Katrin, Batesville, 47006 (US); SCHWADE, Michael, Batesville, 47006 (US)
(74) Representative: Reddie & Grose LLP

(56) References cited:
- US-A1- 2018 130 260
- US-A1- 2019 251 354
- US-A1- 2020 034 622
- US-A1- 2020 210 966
- US-A1- 2020 410 762

## Description

The invention relates to a method of operation of a computer assisted reality system for a medical apparatus, a computer assisted reality system, and a computer program product for executing the method, in particular for E-learning or service utilizations.

Hitherto, learning about and training of an operation of a medical apparatus is done in real surroundings of the medical apparatus by means of a real medical apparatus. However, if such a medical apparatus is already in use, the learning about and training of the operation occupies the medical apparatus so that it is not available for, e.g., surgical interventions. On the other hand, in case that the medical apparatus is not yet available in, e.g., a hospital, access of a training facility is necessary which is not desirable, in particular, during a pandemic crisis.

Moreover, for service or maintenance, in particular, also for service and maintenance training, it is necessary to be present at the medical apparatus and to have access to the documents necessary for the service or maintenance. For determining a state of the medical apparatus or of components thereof, it is necessary to open or dismantle the medical apparatus. In some cases, several service calls are necessary since, in particular in a service case, necessary spare parts are not immediately available.

Therefore, the object underlying the invention is to improve a proper handling with the medical apparatus by improving the learning about and the training of the operation of the medical apparatus and by enhancing a situation in maintenance and service calls.

US 2020/0210966 discloses an apparatus to provide an image modality maintenance care package are disclosed. An example apparatus includes a solution predictor to predict a solution for servicing an imaging device based on the at least one of an error code or an identified issue and information related to previous solutions corresponding to the at least one of the error code or the identified issue. The apparatus further includes a care package generator to generate a customized data structure corresponding to the solution, the customized data structure including automated solutions to service the imaging device. The apparatus further includes an interface to transmit, using a wireless communication, the customized data structure to at least one of the imaging device or a repair device connected to the imaging device and in response to the execution of the automated solutions by the imaging device, obtaining a response corresponding to the servicing of the imaging device using the repair device. The apparatus further includes an information updater to update at least one of the accessed information, the predicted solution, the relevant information, or a digital twin of the imaging device to update subsequent customized care package generation based on the response.

US 2020/0034622 discloses a system for displaying information to a user. The system includes a building management system (BMS) including at least one operating device. The system includes a mixed reality (MR) device. The MR device includes an optical projection system configured to display images and includes a controller including a processing circuit in communication with the optical projection system, the BMS, and a cloud database. The processing circuit is configured to receive a user input from a component of the MR device and provide a request for a device model and data describing the at least one operating device to the cloud database storing the device model and the data. The request is based on the user input. The processing circuit is configured to receive the device model and the data and display, via the optical projection system, a visualization of the device model and the data.

US 2020/0410762 discloses a system, method, and computer-readable medium for modeling and diagnosing a system. System identification information captured by an AR system is used to identify the system and one or more of a model of the components of the system or an overlay of the system is retrieved from a repository or generated based on component identification information or component performance data and sent to the AR system. A composite view of the model or overlay relative to a dynamic image or model of the system allows a technician to visualize the system and components and diagnose the operation of the system without opening the system.

US 2018/0130260 discloses an industrial visualization system that generates and delivers virtual reality (VR) and augmented reality (AR) presentations of industrial facilities to wearable appliances to facilitate remote or enhanced interaction with automation systems within the facility. VR presentations can comprise three-dimensional (3D) holographic views of a plant facility or a location within a plant facility. The system can selectively render a scaled down view that renders the facility as a 3D scale model, or as a first person view that renders the facility as a full scale rendition that simulates the user's presence on the plant floor. The system can also render workflow presentations that guide users through the process of correcting detected maintenance issues.

US 2019/0251354 discloses a head-mounted display device that interfaces with a medical device configured to perform an invasive procedure on a patient. The display device includes a frame for mounting on a person's head, a display, a wireless transceiver configured to communicate with a network, and a processing circuit. The processing circuit is coupled to the frame, the display and the wireless transceiver and receives input data relating to the medical device. The processing circuit retrieves from a memory an instruction relating to the medical device based on the input data and displays the instruction relating to medical device on the display.

The object is achieved by a method according to claim 1, a computer assisted reality system according to claim 13, and a computer program product according to claim 15.

Advantageous further developments are included in the dependent claims.

According to an aspect of the present disclosure, there is provided a method of operation for a computer assisted reality system for a medical apparatus, wherein the computer assisted reality system comprises the medical apparatus comprising a controller and sensors; a remote device; a smart device having a camera and comprising a transmission and reception device for transmitting data to and receiving data from the remote device and the medical apparatus; and an illustration device for illustrating an image, the method comprises the steps: determining a type of the medical apparatus by detecting an input of the type to the smart device or by evaluating an image of a real medical apparatus captured by the camera; determining, with the controller, state variables; collecting, with the controller, detected values of the sensors; exchanging data with the remote device and with the medical apparatus by the transmission and reception device, the data including the state variables and the sensor values; displaying information concerning the medical apparatus via the illustration device, the information comprising a fault message when at least one of the state variables and/or one of the detected values of sensors of the medical apparatus are beyond an allowable range; transmitting the fault message to the remote device; receiving data of a response to the fault message from the remote device by the smart device; and displaying the response via the illustration device.

The computer assisted reality system comprising the smart device enables watching the medical apparatus via the smart device, e.g., smart glasses, a smart phone or a tablet, and displaying information concerning the medical apparatus via the smart device. For performing this task, a computer program product, a so-called application, is executed by the smart device.

For displaying the information concerning the medical apparatus via the illustration device of the smart device, at least a type of the medical apparatus has to be determined. This can be done by the input of the type to the smart device or by the evaluation of the image captured by the camera.

By these steps, the basis for learning about and training of the medical apparatus and for enhanced maintenance or service calls, i.e., the opportunity for input of according instructions, is given so that the proper handling is improved.

By this transmission and reception device, current data can be transmitted from the medical apparatus to the smart device, and data and instructions can be transmitted from the smart device to the medical apparatus.

These state variables can be, for example, a height or a displacement of a tabletop of a surgical table as the medical apparatus so that a current posture of the tabletop can be determined. Furthermore, the state variables can relate to a time-dependent predictive maintenance alert so that the proper handling of, e.g., the surgical table is enhanced.

The detected values of the sensors can indicate, e.g., whether a current overload of the surgical table as the medical apparatus or of components of the surgical table happens. Furthermore, the sensor values can relate to internal states, such as temperatures of electric drives or mechanical loads to any component, and/or to time of operation so that, e.g., a history can be recorded and predictive maintenance alert can be initiated or the states can be evaluated for analysis and complaint documentation.

By this transmission and reception device, current data can be transmitted from the remote device to the smart device, and instructions can be transmitted from the smart device to the remote device which can be, e.g., a data base or an automated service bot. Further, this characteristic enables remote service by a distant diagnostic.

When the fault message is transmitted to the remote device, e.g., faults of the same type of medical apparatus can be evaluated by a service ticketing system which may be linked to a complaint management system implemented in the remote device and, as the case may be, the evaluation can assist in providing a complaint documentation and in evaluating root causes.

By displaying the response of the remote system via the illustration device, more exact data can be provided since fault messages of a plurality of the medical apparatuses of same type may be considered, in particular, for the preventive maintenance alert or for complaint management.

In an advantageous implementation of the method, the computer assisted reality system is formed by an augmented reality system and the illustration device is formed by a touchscreen illustrating an image captured by the camera, wherein the input to the smart device is performed via touching the touchscreen by an object, wherein the method comprises the step: illustrating an illustration of the medical apparatus according to the type of the medical apparatus against a backdrop via the illustration device.

By the use of the augmented reality system, the execution of the method can be done with manageable efforts since merely a smart device, such as a tablet computer or a cell phone, is necessary for improving the proper handling of the medical apparatus. In the augmented reality system, the image of a real world can be superimposed with additional information on the display of the smart device. Moreover, the smart device captures images by its camera for illustrating the medical apparatus against the backdrop via the illustration device of the smart device and inputs can be performed by touching the touchscreen by the object, e.g., a finger.

According to an advantageous implementation of the method, it further comprises the steps: capturing an image of surroundings of the smart device by the camera, and illustrating an illustration of a 3D model of the type of the medical apparatus in the image of the surroundings being the backdrop via the illustration device.

When the image of the surroundings of the smart devices captured by the camera and illustrated by the illustration device and, also, the illustration of the 3D model of the type of the medical apparatus are displayed, the medical apparatus can be illustrated in learner's surroundings. This can be either a real operating place in an operating theater of a hospital or, e.g., in a room of a training facility or in the home of a person. The 3D model is then displayed in quasi-real operating conditions so that the proper handling of the medical apparatus is improved.

In another advantageous implementation of the method, it further comprises the steps: capturing an image of the medical apparatus by the camera, illustrating the captured image of the real medical apparatus against the backdrop, and determining the type of the medical apparatus by evaluating the captured image of the real medical apparatus.

By this implementation, a manual determination of the type of the medical apparatus is not necessary since, from the captured image of the real medical apparatus, its type can be unambiguously determined. Moreover, the medical apparatus in its actual configuration can be determined and further information resulting thereof can be displayed.

In another advantageous implementation of the method, the computer assisted reality system is formed by a mixed reality system and the illustration device is formed by smart glasses, wherein the input to the smart device is performed via capturing an object and a motion of the object by the camera, wherein the method comprises the step: illustrating an illustration of a 3D model of the type of the medical apparatus via the illustration device.

Due to the use of the mixed reality system comprising the smart glasses which enables watching the real surrounding and illustrating the 3D model of the type of the medical apparatus as a hologram, a more sophisticated impression of the medical apparatus can be achieved and a more exact handling of the medical apparatus is possible since a user virtually touches and operates the medical apparatus almost as in real environments by moving his fingers or hands. In the mixed reality system, the holograms can be placed in a real world so that they seem to be actually present in the observed area. By means of the smart glasses, the real items of an area including the hands of the operator of the system as well as the holograms illustrating desired further items can be observed and the input can be performed by detecting the object, e.g., the hand or fingers.

In a further advantageous implementation of the method, the method further comprises the step: moving the illustration of the 3D model of the type of the medical apparatus by virtually touching the illustration of the 3D model of the type of the medical apparatus by means of an object and by moving the object.

Due to these options, the illustration of the medical apparatus can be moved, e.g., by means of a finger or a hand on the touch screen or captured by the camera, and can be regarded from different sides. Depending on the available degrees of freedom, the medical apparatus can be displaced and/or rotated in order to have the opportunity to regard also views of the medical apparatus which, in real life, would only be possible when the operator himself would move so that the proper handling of the medical apparatus is improved.

According to a further advantageous implementation of the method, the information comprises a virtual keypad of the medical apparatus, and the method further comprises the step: actuating motions of the 3D model of the medical apparatus according to inputs to the virtual keypad.

The virtual keypad of the medical apparatus enables an operation according to the operation of an actual medical apparatus. In particular, all or a predetermined selection of the functions of the actual medical apparatus can be performed by operating the virtual keypad. The 3D model of the medical apparatus executes the instructed functions. Therefore, a remote E-learning is possible.

In another advantageous implementation of the method, it further comprises the steps: capturing an image of the medical apparatus by the camera, and determining the type of the medical apparatus by evaluating the captured image of the real medical apparatus.

By this implementation, a manual determination of the type of the medical apparatus is not necessary since, from the captured image of the real medical apparatus, its type can be unambiguously determined. Moreover, the medical apparatus in its actual configuration can be determined and further information resulting thereof can be displayed.

According to a further advantageous implementation of the method, the information comprises a 3D model of a subassembly of the medical apparatus or of a component of the medical apparatus, and the method further comprises the step: rotating the 3D model of the subassembly or of the component by virtually touching the 3D model of the subassembly or of the component by means of an object and by moving the object.

By this characteristic, in case of maintenance or of a service call, components of the medical apparatus can easily be identified by visually investigating them from different views without lavishly opening the physical medical apparatus.

According to a further advantageous implementation of the method, the information comprises at least one of a service manual and a user manual of the medical apparatus, and the method further comprises the step: selecting an area in the service manual or the user manual by virtually touching the area by means of an object, and, thereby, providing further data about this area.

When selecting a specific area in the service manual, the further data can be, e.g., a video for assembling or dismantling a subassembly or instructions concerning, e.g., screw torques. The further data when selecting areas in the user manual can for example be videos of certain functions of the medical apparatus or instructions for attaching or detaching further components of the medical apparatus. Due to these data, maintenance and service calls or operation of the medical apparatus can be facilitated and the learning about the medical apparatus is improved.

According to a further advantageous implementation of the method, the information comprises the virtual keypad of the medical apparatus, and the method further comprises the step: actuating motions of the real medical apparatus according to inputs to the virtual keypad via the transmission and reception device.

The virtual keypad of the medical apparatus enables an operation corresponding to the operation of an actual medical apparatus. In particular, all of the functions of the actual medical apparatus can be performed by operating the virtual keypad and the real table performs these functions. Therefore, in case of a maintenance or service call, the medical apparatus can easily be operated by the smart device.

In a further advantageous implementation of the method, the information comprises an identifier of the medical apparatus, the identifier comprises at least one of the serial number of the medical apparatus and a firmware release of the software, the identifier is either input to the smart device or detected by evaluating the data transmitted from the medical apparatus, and the smart device retrieves data according to the identifier from a memory of the smart device or from the medical apparatus.

By being informed about the serial number of the medical apparatus or the firmware release of the software by the smart device, an exact determination of the functions of the medical apparatus or a determination of components or subassemblies is easily possible by retrieving the according data.

The information comprises at least one of a 3D model of a subassembly of the medical apparatus and a bill of material of the subassembly, and the method further comprises the steps: selecting a component of the subassembly by virtually touching the component in the 3D model of the subassembly or in the bill of material of the subassembly by means of an object, and transmitting a request concerning the selected component to the remote device by the smart device.

By this function, components of the medical apparatus can be selected easily for, e.g., ordering a spare part or for gathering additional data about this component.

According to a further aspect of the invention, a computer assisted reality system comprises a medical apparatus comprising a controller and sensors, a remote device, and a smart device having a camera and comprising a transmission and reception device for transmitting data to and receiving data from the remote device and the medical apparatus, and an illustration device for illustrating an image, wherein the computer assisted reality system is configured to execute a method for operation of the computer assisted reality system for the medical apparatus.

By this computer assisted reality system, the basis for learning about and training of the medical apparatus and for enhanced maintenance or service calls, i.e., the opportunity for input of according instructions, is given so that the proper handling is improved.

In an advantageous implementation of the computer assisted reality system, the medical apparatus is formed by a surgical table.

The surgical table has plenty of functions which partially depend on each other so that training of the operation of the surgical table is an important measure for the proper handling during use and, also, due to the complexity of the structure of the surgical table, assistance in maintenance and service improves the proper handling in this situation.

According to another aspect of the invention, a computer program product is configured to perform a method for operating of the augmented reality system for the medical apparatus.

By this computer program product, the basis for learning about and training of the medical apparatus and for enhanced maintenance or service calls, i.e., the opportunity for input of according instructions, is given so that the proper handling is improved.

Subsequently, the invention is elucidated by means of embodiments referring to the attached drawings.

In particular,
- Fig. 1: shows a structural view of an augmented reality system as an embodiment of a computer assisted reality system according to the invention;
- Fig. 2: shows an image of a 3D model of a surgical table as a medical apparatus and of a virtual keypad on a touchscreen of a smart device;
- Fig. 3: shows smart glasses as a further embodiment of the computer assisted reality system; and
- Fig. 4: shows a flowchart of a method according to the invention.

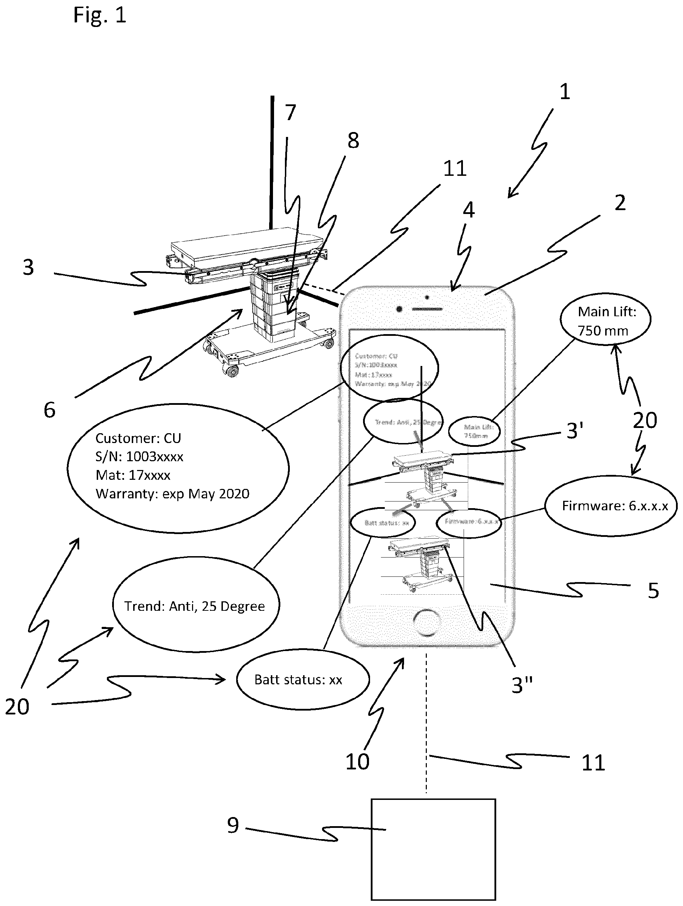

**Fig. 1** shows an augmented reality system 1 as an embodiment of a computer assisted reality system. The augmented reality system 1 comprises a smart device 2 and a surgical table 3 as the medical apparatus. In alternative embodiments, the medical apparatus is formed by, e.g., a surgical lamp, a surgical camera system or medical ceiling mounted support systems.

The smart device 2 comprises a camera 4 and a touchscreen 5 as an illustration device. Moreover, the smart device 2 is provided with a processor configured to execute a computer program performing a method for operation of the augmented reality system 1. This method enables an operator to use or investigate the surgical table 3 supported by images captured by the camera 4. At least one of the surgical table 3 and a backdrop is captured by the camera 4 and, therefore, a quasi-real situation is displayed on the touchscreen 5.

On the touchscreen 5, an image captured by the camera 4 is shown. In particular, an image of surroundings of the smart device 2 is captured by the camera 4 and displayed as a backdrop on the touchscreen 5. In this case, a corner 6 of a room is shown as the surroundings. Additionally or alternatively, an image of a real one of the surgical table 3' captured by the camera 4 is displayed and, moreover, an illustration of a 3D model of a type of the surgical table 3" is shown against the backdrop on the touchscreen 5. The image of the real one of the surgical table 3' can be displayed either against the real surroundings as the backdrop or, e.g., in a white frame as the backdrop.

Except from the images of the surgical table 3, information 20 concerning the surgical table 3 is displayed on the touchscreen 5. In the shown example, elements of information 20, such as an indication of a customer ("Customer"), an identifier, i.e., in this case, a serial number ("S/N"), a material number ("Mat") identifying the type of the surgical table 2, and an expiration date of the warranty ("Warranty"), are displayed. A further information 20 is the indication of the release of the firmware ("Firmware").

Furthermore, the touchscreen 5 is configured for input of instructions or data by touching the touch screen 5 by an object, e.g., a finger.

The surgical table 3 comprises a controller 7 and sensors 8 detecting various states of the surgical table 3. In the shown embodiment, at least a sensor 8 detecting a battery status is provided. The detected value of the sensor ("Batt status") is displayed on the touchscreen 5.

On the touchscreen 5, various state variables determined by the controller 7, such as a variable indicating the tilting ("Trend"), in the shown embodiment, 25° in the Anti-Trendelenburg direction, and an extension of a column of the surgical table 3 ("Main Lift"), as 750 mm, are displayed.

In alternative embodiments, not all of these elements of information 20 are displayed and/or other elements of information 20 are displayed on the touchscreen 5.

Moreover, a remote device 9 is provided and the smart device 2 comprises a transmission and reception device 10. The transmission and reception device 10 provides a respective transmission path 11 for transmitting data to and receiving data from, on the one hand, the surgical table 3 and, on the other hand, the remote device 9. In alternative embodiments, the transmission path 11 is only either provided for data transmission between the smart device 2 and the surgical table 3 or between the smart device 2 and the remote device 9.

In use, the augmented reality system 1 provides the opportunity to virtually operate or investigate the surgical table 3 as the medical apparatus based on an image captured by the camera 4 of the smart device 2.

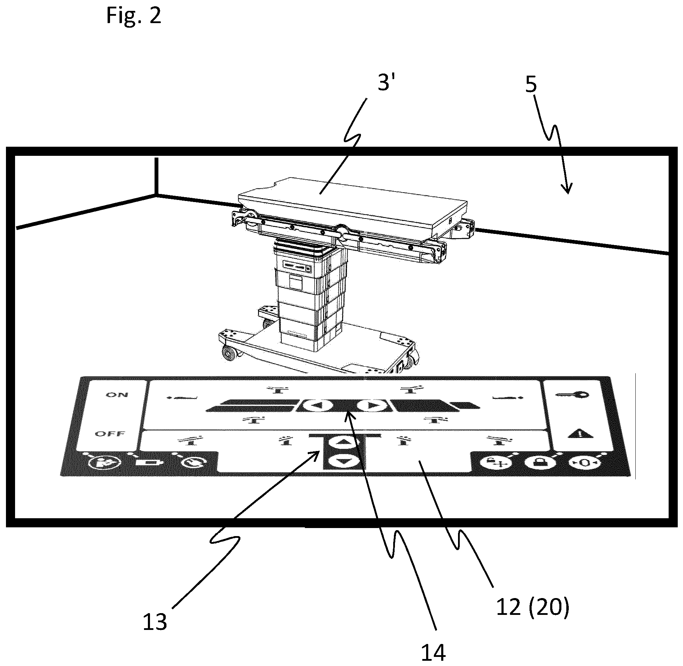

**Fig. 2** shows an image of the 3D model of the surgical table 3' as the medical apparatus and of a virtual keypad 12 on the touchscreen 5.

The virtual keypad 12 comprises keys for actuating motions of the shown image of the 3D model of the surgical table 3'. The motions are actuated according to inputs to the virtual keypad 12, i.e., according to the touched virtual keys. In this embodiment, keys 13 for a height adjustment of the tabletop of the surgical table 3 are provided. Furthermore, further keys 14 for a horizontal displacement of the tabletop of the image of the 3D model of the surgical table 3' in its longitudinal direction are provided.

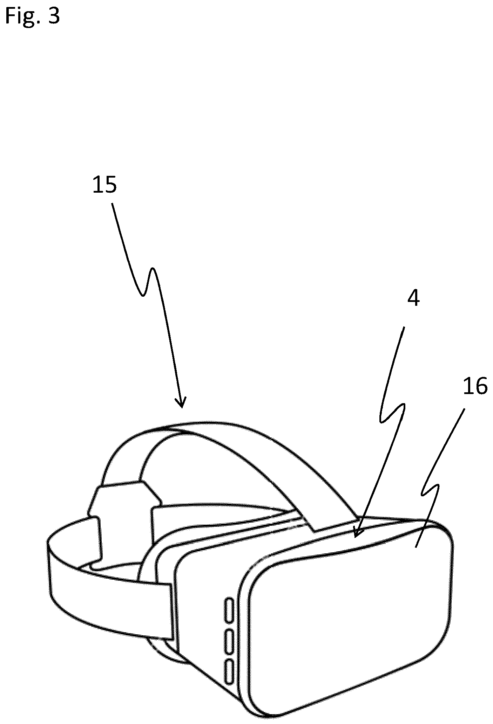

**Fig. 3** shows smart glasses 15 as a smart device of further embodiment of a computer assisted reality system in the form of a mixed reality system.

The smart glasses 15 comprise a screen 16 through which the surroundings can be observed by seeing through the screen 16. In the screen 16, a layer is provided as the illustration means for illustrating the 3D model of the surgical table 3' and the information in the manner of a head-up display. Further, the smart glasses 15 comprise the camera 4. In an alternative embodiment, another structure of the screen is possible.

The mixed reality system distinguishes from the augmented reality system in that it does not comprise the touch screen 5 by which images captured by the camera 4 are illustrated and by which input to the smart device is performed. Instead, inputs can be received by capturing the object and a motion of the object by the camera 4. The object, i.e., the finger or a hand, approaches the virtually illustrated 3D model of the type of the medical apparatus and either moves it by virtually touching the illustration of the 3D model or performs other activities, such as actuating a function via the virtual keypad.

The information corresponds to the information described with respect to the augmented reality system. Also, the transmission and reception device according 10 to that of the augmented reality system is provided.

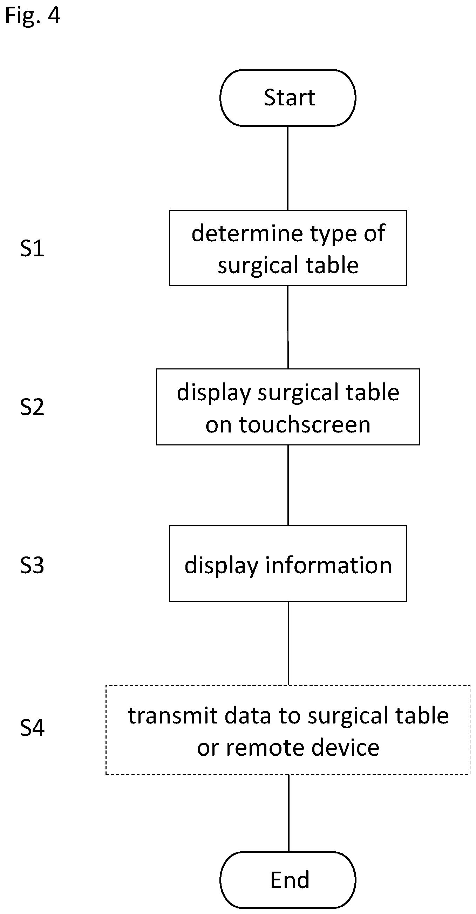

**Fig. 4** shows a flowchart of a method according to the invention.

In use of the augmented reality system 1 as the computer assisted reality system for a virtual operation of the surgical table 3 as the medical apparatus, e.g., for E-learning or for a service diagnostic, in a first step S1, the type of the surgical table 3 is determined. This determination happens by input of the type of the surgical table 3 by means of the touchscreen 5. Alternatively, the type of the surgical table 3 can also be determined by evaluating an image of the surgical table 3 captured by the camera 4 or by evaluating data received by the smart device 2 from the surgical table 3 via the transmission path 11.The evaluation of the image of the surgical table 3 takes place by comparing specific characteristics of the captured surgical table 3 with characteristics stored in a data base, e.g., in a memory of the smart device 2.

As the case may be, the image of the surroundings of the smart device 2 is captured as the backdrop and the image of the real surgical table 3 is captured. Alternatively, an image of merely one of the surroundings and the surgical table 3 is captured and the image of the real surgical table is illustrated against virtual surroundings or a virtual surgical table is illustrated against an image of the real surroundings.

In a second step S2, an image of the surgical table 3 according to the type of the surgical table 3 is displayed on the touchscreen 5. In particular, either the image of the real one of the surgical table 3' captured by the camera 4 is displayed or the image of the 3D model of the type of the surgical table 3" or both of the images are displayed against the backdrop. Alternatively, the real backdrop is not displayed but the image of the 3D model is shown in a white frame.

The image of the 3D model of the type of the surgical table 3" can be moved on the touchscreen 5 by touching the image of the 3D model of the type of the surgical table 3" on the touchscreen 5 by means of the object, e.g., the finger, and by moving the object on the touchscreen 5. Depending on the available degrees of freedom, the image of the 3D model of the surgical table 3" can be displaced and/or rotated on the touchscreen 5. Moreover, optionally, the image of the 3D model of the surgical table 3" can be up-scaled or downscaled.

Furthermore, in step S3, the information 20 concerning the surgical table 3 is displayed on the touchscreen 5.

The displayed information 20 comprise the virtual keypad 12, a 3D model of at least one of a subassembly of the surgical table 3 and a component of the surgical table 3, a bill of material of a subassembly of the surgical table 3, the service manual of the surgical table 3, the user manual of the surgical table 3, at least one of the identifiers of the surgical table 3, at least one of the state variables of the surgical table 3, at least one of the detected value of the sensors 8, and a fault message. In alternative embodiments, not all of these elements of the information 20 are displayed or further elements of the information 20 are displayed on the touchscreen 5.

Optionally, the data of the smart device 2 is transmitted to or exchanged with the surgical table 3 or transmitted to or exchanged with the remote device 9 by the transmission and reception device 10. The remote device 9 is a network, such as the Internet, wherein various services are offered in the network. Alternatively, the remote device 9 may be a single computer or a local network.

The virtual keypad 12 is used for actuating motions of the 3D model of the surgical table 3" according to inputs to the virtual keypad 12. By touching corresponding symbols of the virtual keypad 12, the motions of the 3D model of the surgical table 3" are actuated. Optionally, the real surgical table 3 can be controlled such that motions of the real surgical table 3 are actuated according to the inputs to the virtual keypad 12.

The displayed 3D model of the at least one of a subassembly of the surgical table 3 and a component of the surgical table 3 can be rotated on the touchscreen 5 by touching this 3D model on the touchscreen 5 by means of the object and by moving the object.

When selecting a component of the subassembly of the surgical table 3 by touching the touchscreen 5 by means of the object, a request concerning the selected component is transmitted to the remote device 9. This may be an order for a spare part of this component. The component can be selected by touching either the 3D model including the component or a line in the bill of material.

In the displayed service manual or user manual of the surgical table 3, an area thereof is selected by touching by means of the object. By selecting this area, further data about this area can be provided. This is, e.g., a video for assembling or disassembling a selected subassembly or further data or instructions concerning this subassembly. Alternatively, other data about this area is provided.

From the displayed identifier which, in the shown embodiment, includes the serial number of the surgical table 3 and a software release of the software of the surgical table 3, the surgical table can unambiguously be identified by the smart device 2.

Alternatively, only one or another identifier is indicated. This identifier is either input to the touchscreen, e.g., by means of a virtual numerical or alphanumerical key panel, or it is detected by evaluating the data transmitted from the surgical table. Then, the smart device 2 retrieves data according to the identifier from a memory of the smart device 2, from the surgical table 3, or from the remote device 9.

Displaying of the state variables enables, for example, an exact determination of a posture of the tabletop of the surgical table 3. Moreover, the state variables may be based on evaluated working conditions and a date of a next preventive maintenance can be indicated thereof.

Displaying of detected value of sensors transmitted to the smart device 2 enables a detection of current working conditions, such as a temperature of an electric motor or a battery status.

If a fault occurs, i.e., if at least one of a critical state variable or a detected value of a critical sensor is beyond an allowable range, the fault message is displayed on the touchscreen 5. Such a fault message can also be signaled by means of an acoustic signal. This fault message is also transmitted to the remote device 9. In an alternative embodiment, the fault message is only displayed by the touchscreen 5.

The smart device 2 may receive data of a response to the fault message sent to the remote device 9 from the remote device 9. This response is displayed on the touchscreen 5. Such a response is generated, e.g., by an automated service bot or help desk, when an appropriate countermeasure for, e.g., remedying a fault is available. Moreover, if a problem cannot be resolved by the service bot, based on the state variable or value of a critical sensor transmitted to the remote device 9, a service case can be escalated and human service operators can deal with the problem online.

In case of such a fault or any technical problem, a group chat communication between technicians is possible by transmitting and sharing files, firmware, images, or videos via the smart device 2.

While the invention has been illustrated and described in detail in the drawings and the foregoing description, such illustration and description are to be considered illustrative or exemplary and not restrictive. The invention is not limited to the disclosed embodiments, but defined only by the claims. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. A method of operation for a computer assisted reality system (1) for a medical apparatus, wherein the computer assisted reality system (1) comprises:
the medical apparatus (3) comprising a controller (7) and sensors (8);
a remote device (9);
a smart device (2) having a camera (4) and comprising a transmission and reception device (10) for transmitting data to and receiving data from the remote device (9) and the medical apparatus (3); and
an illustration device (5) for illustrating an image;
the method comprising the steps:
determining, by the smart device (2), a type of the medical apparatus (3) by detecting an input of the type to the smart device (2) or by evaluating an image of a real medical apparatus captured by the camera (4);
determining, with the controller (7), state variables;
collecting, with the controller (7), detected values of the sensors (8);
exchanging data with the remote device (9) and with the medical apparatus (3) by the transmission and reception device (10), the data including the state variables and the sensor values;
displaying, by the smart device (2), information (20) concerning the medical apparatus via the illustration device, the information (20) comprising a fault message when at least one of the state variables and/or one of the detected values of sensors (8) of the medical apparatus are beyond an allowable range;
transmitting, by the smart device (2), the fault message to the remote device (9);
receiving data of a response to the fault message from the remote device (9) by the smart device (2); and
displaying, by the smart device (2), the response via the illustration device, and wherein the information (20) comprises at least one of a 3D model of a subassembly of the medical apparatus and a bill of material of the subassembly, and
the method further comprises the steps:
selecting a component of the subassembly by virtually touching the component in the 3D model of the subassembly or in the bill of material of the subassembly by means of an object, and
transmitting a request concerning the selected component to the remote device (9) by the smart device (2).

2. The method of claim 1, wherein the computer assisted reality system is formed by an augmented reality system and the illustration device is formed by a touchscreen (5) illustrating an image captured by the camera (4), wherein
the input to the smart device is performed via touching the touchscreen (5) by an object, comprising the step:
illustrating an illustration of the medical apparatus according to the type of the medical apparatus against a backdrop via the illustration device.

3. The method of claim 2, further comprising the step:
capturing an image of surroundings of the smart device (2) by the camera (4); and
illustrating an illustration of a 3D model of the type of the medical apparatus in the image of the surroundings being the backdrop via the illustration device.

4. The method of claim 3, further comprising the steps:
capturing an image of the medical apparatus by the camera (4);
illustrating the captured image of the real medical apparatus against the backdrop; and
determining the type of the medical apparatus by evaluating the captured image of the real medical apparatus.

5. The method of claim 1, wherein
the computer assisted reality system is formed by a mixed reality system and the illustration device is formed by smart glasses (15),
the input to the smart device is performed via capturing an object and a motion of the object by the camera (4), and the method comprises the step:
illustrating an illustration of a 3D model of the type of the medical apparatus via the illustration device.

6. The method of any one of claims 3 to 5, further comprising the step:
moving the illustration of the 3D model of the type of the medical apparatus by virtually touching the illustration of the 3D model of the type of the medical apparatus via the illustration device by means of the object and by moving the object.

7. The method of any one of claims 3 to 6, wherein
the information (20) comprises a virtual keypad (12) of the medical apparatus, and
the method further comprises the step:
actuating motions of the 3D model of the medical apparatus according to inputs to the virtual keypad (12).

8. The method of any preceding claim, further comprising the steps:
capturing an image of the medical apparatus by the camera (4); and
determining the type of the medical apparatus by evaluating the captured image of the real medical apparatus.

9. The method of any preceding claim, wherein
the information (20) comprises a 3D model of a subassembly of the medical apparatus or of a component of the medical apparatus, and
the method further comprises the step:
rotating the 3D model of the subassembly or of the component illustrated by the illustration device by virtually touching the 3D model of the subassembly or of the component via the illustration device by means of an or the object and by moving the object.

10. The method of any preceding claim, wherein
the information (20) comprises at least one of a service manual and a user manual of the medical apparatus, and
the method further comprises the step:
selecting an area in the service manual or the user manual by virtually touching the area by means of an or the object, and, thereby, providing further data about this area.

11. The method of any preceding claim, wherein
the information (20) comprises a or the virtual keypad (12) of the medical apparatus, and
the method further comprises the step:
actuating motions of the real medical apparatus according to inputs to the virtual keypad (12) via the transmission and reception device (10).

12. The method of any preceding claim, wherein
the information (20) comprises an identifier of the medical apparatus,
the identifier comprises at least one of a serial number of the medical apparatus and a firmware release of the software,
the identifier is either input to the smart device or detected by evaluating data transmitted from the medical apparatus, and
the method further comprises the step:
retrieving data according to the identifier from a memory of the smart device (2) or from the medical apparatus by the smart device (2) by means of the transmission and reception device (10).

13. A computer assisted reality system (1) comprising a medical apparatus (3) comprising a controller (7) and sensors (8), a remote device (9), and a smart device (2) having a camera (4) and comprising a transmission and reception device (10) for transmitting data to and receiving data from the remote device (9) and the medical apparatus (3), and an illustration device for illustrating an image, wherein the computer assisted reality system (1) is configured to execute a method according to any one of the preceding claims.

14. The computer assisted reality system of claim 13, wherein
the medical apparatus is formed by a surgical table (3).

15. A computer program product configured to perform a method according to any one of the claims 1 to 12.

## Patentansprüche

1. Verfahren zum Betreiben eines computergestützten Reality-Systems (1) für eine medizinische Vorrichtung, wobei das computergestützte Reality-System (1) Folgendes umfasst:
die medizinische Vorrichtung (3) mit einer Steuerung (7) und Sensoren (8);
ein Remote-Gerät (9);
ein Smart-Gerät (2) mit einer Kamera (4) und einem Sende- und Empfangsgerät (10) zum Senden von Daten zu und und Empfangen von Daten von dem Remote-Gerät (9) und der medizinischen Vorrichtung (3); und
ein Darstellungsgerät (5) zum Darstellen eines Bildes;
wobei das Verfahren die folgenden Schritte beinhaltet:
Bestimmen, durch das Smart-Gerät (2), eines Typs der medizinischen Vorrichtung (3) durch Erkennen einer Eingabe des Typs in das Smart-Gerät (2) oder durch Auswerten eines von der Kamera (4) aufgenommenen Bildes einer realen medizinischen Vorrichtung;
Bestimmen von Zustandsvariablen mit der Steuerung (7);
Sammeln von erfassten Werten der Sensoren (8) mit der Steuerung (7);
Austauschen von Daten mit dem Remote-Gerät (9) und mit der medizinischen Vorrichtung (3) durch das Sende- und Empfangsgerät (10), wobei die Daten die Zustandsvariablen und die Sensorwerte umfassen;
Anzeigen, durch das Smart-Gerät (2), von Informationen (20) über die medizinische Vorrichtung über das Darstellungsgerät, wobei die Informationen (20) eine Fehlermeldung umfassen, wenn mindestens eine der Zustandsvariablen und/oder einer der erfassten Werte von Sensoren (8) der medizinischen Vorrichtung jenseits eines zulässigen Bereichs liegen;
Senden, durch das Smart-Gerät (2), der Fehlermeldung zu dem Remote-Gerät (9);
Empfangen von Daten einer Antwort auf die Fehlermeldung von dem Remote-Gerät (9) durch das Smart-Gerät (2); und
Anzeigen, durch das Smart-Gerät (2), der Antwort über das Darstellungsgerät, wobei die Informationen (20) ein 3D-Modell einer Unterbaugruppe der medizinischen Vorrichtung und/oder eine Stückliste der Unterbaugruppe umfassen, und
wobei das Verfahren ferner die folgenden Schritte beinhaltet:
Auswählen einer Komponente der Unterbaugruppe durch virtuelles Berühren der Komponente im 3D-Modell der Unterbaugruppe oder in der Stückliste der Unterbaugruppe mittels eines Objekts, und
Senden einer Anforderung bezüglich der ausgewählten Komponente an das Remote-Gerät (9) durch das Smart-Gerät (2).

2. Verfahren nach Anspruch 1, wobei das computergestützte Reality-System durch ein Augmented-Reality-System gebildet wird und das Darstellungsgerät durch einen Touchscreen (5) gebildet wird, der ein von der Kamera (4) aufgenommenes Bild darstellt, wobei
die Eingabe in das Smart-Gerät durch Berühren des Touchscreens (5) mit einem Objekt erfolgt, das den folgenden Schritt beinhaltet:
Darstellen einer Darstellung der medizinischen Vorrichtung gemäß dem Typ der medizinischen Vorrichtung vor einem Hintergrund über das Darstellungsgerät.

3. Verfahren nach Anspruch 2, das ferner den folgenden Schritt beinhaltet:
Aufnehmen eines Bildes der Umgebung des Smart-Geräts (2) durch die Kamera (4); und
Darstellen einer Darstellung eines 3D-Modells des Typs der medizinischen Vorrichtung in dem Bild der Umgebung, das den Hintergrund bildet, über das Darstellungsgerät.

4. Verfahren nach Anspruch 3, das ferner die folgenden Schritte beinhaltet:
Aufnehmen eines Bildes der medizinischen Vorrichtung durch die Kamera (4);
Darstellen des aufgenommenen Bildes der realen medizinischen Vorrichtung vor dem Hintergrund; und
Bestimmen des Typs der medizinischen Vorrichtung durch Auswerten des aufgenommenen Bildes der realen medizinischen Vorrichtung.

5. Verfahren nach Anspruch 1, wobei
das computergestützte Reality-System durch ein Mixed-Reality-System gebildet wird und das Darstellungsgerät durch eine Smart-Brille (15) gebildet wird,
die Eingabe in das Smart-Gerät durch Aufnehmen eines Objekts und einer Bewegung des Objekts durch die Kamera (4) erfolgt und das Verfahren den folgenden Schritt beinhaltet:
Darstellen einer Darstellung eines 3D-Modells des Typs der medizinischen Vorrichtung über das Darstellungsgerät.

6. Verfahren nach einem der Ansprüche 3 bis 5, das ferner den folgenden Schritt beinhaltet:
Bewegen der Darstellung des 3D-Modells des Typs der medizinischen Vorrichtung durch virtuelles Berühren der Darstellung des 3D-Modells des Typs der medizinischen Vorrichtung über das Darstellungsgerät mit dem Objekt und durch Bewegen des Objekts.

7. Verfahren nach einem der Ansprüche 3 bis 6, wobei
die Informationen (20) eine virtuelle Tastatur (12) der medizinischen Vorrichtung umfassen und
das Verfahren ferner den folgenden Schritt beinhaltet:
Auslösen von Bewegungen des 3D-Modells der medizinischen Vorrichtungen gemäß Eingaben in die virtuelle Tastatur (12).

8. Verfahren nach einem vorherigen Anspruch, das ferner die folgenden Schritte beinhaltet:
Aufnehmen eines Bildes der medizinischen Vorrichtung durch die Kamera (4); und
Bestimmen des Typs der medizinischen Vorrichtung durch Auswerten des aufgenommenen Bildes der realen medizinischen Vorrichtung.

9. Verfahren nach einem vorherigen Anspruch, wobei
die Informationen (20) ein 3D-Modell einer Unterbaugruppe der medizinischen Vorrichtung oder einer Komponente der medizinischen Vorrichtung umfassen, und
das Verfahren ferner den folgenden Schritt beinhaltet:
Drehen des durch das Darstellungsgerät dargestellten 3D-Modells der Unterbaugruppe oder der Komponente durch virtuelles Berühren des 3D-Modells der Unterbaugruppe oder der Komponente über das Darstellungsgerät mit einem oder dem Objekt und durch Bewegen des Objekts.

10. Verfahren nach einem vorherigen Anspruch, wobei
die Informationen (20) ein Wartungshandbuch und/oder ein Benutzerhandbuch der medizinischen Vorrichtung umfassen, und
das Verfahren ferner den folgenden Schritt beinhaltet:
Auswählen eines Bereichs im Wartungshandbuch oder im Benutzerhandbuch durch virtuelles Berühren des Bereichs mit einem oder dem Objekt und dadurch Bereitstellen weiterer Daten über diesen Bereich.

11. Verfahren nach einem vorherigen Anspruch, wobei
die Informationen (20) eine oder die virtuelle Tastatur (12) der medizinischen Vorrichtung umfassen und
das Verfahren ferner den folgenden Schritt beinhaltet:
Auslösen von Bewegungen der realen medizinischen Vorrichtung gemäß Eingaben in die virtuelle Tastatur (12) über das Sende- und Empfangsgerät (10).

12. Verfahren nach einem vorherigen Anspruch, wobei
die Informationen (20) eine Kennung der medizinischen Vorrichtung umfassen,
die Kennung eine Seriennummer der medizinischen Vorrichtung und/oder eine Firmware-Version der Software umfasst,
die Kennung entweder in das Smart-Gerät eingegeben oder durch Auswerten von von der medizinischen Vorrichtung gesendeten Daten erkannt wird, und
das Verfahren ferner den folgenden Schritt beinhaltet:
Abrufen von Daten gemäß der Kennung aus einem Speicher des Smart-Geräts (2) oder aus der medizinischen Vorrichtung durch das Smart-Gerät (2) mittels des Sende- und Empfangsgeräts (10).

13. Computergestütztes Reality-System (1) umfassend eine medizinische Vorrichtung (3) umfassend eine Steuerung (7) und Sensoren (8), ein Remote-Gerät (9) und ein Smart-Gerät (2) mit einer Kamera (4), und umfassend ein Sende- und Empfangsgerät (10) zum Senden von Daten zu und Empfangen von Daten von dem Remote-Gerät (9) und der medizinischen Vorrichtung (3), und ein Darstellungsgerät zum Darstellen eines Bildes, wobei das computergestützte Reality-System (1) zum Durchführen eines Verfahrens nach einem der vorherigen Ansprüche konfiguriert ist.

14. Computergestütztes Reality-System nach Anspruch 13, wobei die medizinische Vorrichtung durch einen Operationstisch (3) gebildet wird.

15. Computerprogrammprodukt, das zum Durchführen eines Verfahrens nach einem der Ansprüche 1 bis 12 konfiguriert ist.

## Revendications

1. Procédé de fonctionnement d'un système de réalité assisté par ordinateur (1) pour un appareil médical, dans lequel le système de réalité assisté par ordinateur (1) comprend :
l'appareil médical (3) comprenant un contrôleur (7) et des capteurs (8) ;
un dispositif à distance (9) ;
un dispositif intelligent (2) ayant une caméra (4) et comprenant un dispositif de transmission et de réception (10) pour transmettre des données au et recevoir des données du dispositif à distance (9) et de l'appareil médical (3) ; et
un dispositif d'illustration (5) pour illustrer une image ;
le procédé comprenant les étapes consistant à :
déterminer, par le dispositif intelligent (2), un type de l'appareil médical (3) en détectant une entrée du type au dispositif intelligent (2) ou en évaluant une image d'un appareil médical réel capturée par la caméra (4) ;
déterminer, avec le contrôleur (7), des variables d'état ;
recueillir, avec le contrôleur (7), des valeurs détectées des capteurs (8) ;
échanger des données avec le dispositif à distance (9) et avec l'appareil médical (3) par le dispositif de transmission et de réception (10), les données comprenant les variables d'état et les valeurs de capteurs ;
afficher, par le dispositif intelligent (2), des informations (20) concernant l'appareil médical via le dispositif d'illustration, les informations (20) comprenant un message d'erreur lorsque au moins l'une des variables d'état et/ou l'une des valeurs détectées des capteurs (8) de l'appareil médical sont au-delà d'une plage admissible ;
transmettre, par le dispositif intelligent (2), le message d'erreur au dispositif à distance (9) ;
recevoir des données en réponse au message d'erreur du dispositif à distance (9) par le dispositif intelligent (2) ; et
afficher, par le dispositif intelligent (2), la réponse via le dispositif d'illustration, et dans lequel les informations (20) comprennent au moins l'un d'entre un modèle 3D du sous-ensemble de l'appareil médical et une nomenclature du sous-ensemble ; et
le procédé comprenant en outre les étapes consistant à :
sélectionner un composant du sous-ensemble en touchant virtuellement le composant dans le modèle 3D du sous-ensemble ou dans la nomenclature du sous-ensemble au moyen d'un objet, et
transmettre une requête concernant le composant sélectionné au dispositif à distance (9) par le dispositif intelligent (2).

2. Procédé selon la revendication 1, dans lequel le système de réalité assisté par ordinateur est formé par un système de réalité augmentée et le dispositif d'illustration est formé par un écran tactile (5) illustrant une image capturée par la caméra (4), dans lequel
l'entrée au dispositif intelligent est effectuée en touchant l'écran tactile (5) avec un objet, comprenant l'étape consistant à :
illustrer une illustration de l'appareil médical conformément au type de l'appareil médical contre une toile de fond via le dispositif d'illustration.

3. Procédé selon la revendication 2, comprenant en outre l'étape consistant à :
capturer une image des alentours du dispositif intelligent (2) avec la caméra (4) ; et
illustrer une illustration d'un modèle 3D du type de l'appareil médical dans l'image des alentours étant la toile de fond via le dispositif d'illustration.

4. Procédé selon la revendication 3, comprenant en outre les étapes consistant à :
capturer une image de l'appareil médical avec la caméra (4) ;
illustrer l'image capturée de l'appareil médical réel contre la toile de fond ; et
déterminer le type de l'appareil médical en évaluant l'image capturée de l'appareil médical réel.

5. Procédé selon la revendication 1, dans lequel
le système de réalité assisté par ordinateur est formé par un système de réalité mixte et le dispositif d'illustration est formé par des lunettes intelligentes (15),
l'entrée au dispositif intelligent est effectuée en capturant un objet et un mouvement de l'objet avec la caméra (4), et le procédé comprend l'étape consistant à :
illustrer une illustration d'un modèle 3D du type de l'appareil médical via le dispositif d'illustration.

6. Procédé selon l'une quelconque des revendications 3 à 5, comprenant en outre l'étape consistant à :
déplacer l'illustration du modèle 3D du type de l'appareil médical en touchant virtuellement l'illustration du modèle 3D du type de l'appareil médical via le dispositif d'illustration au moyen de l'objet et en déplaçant l'objet.

7. Procédé selon l'une quelconque des revendications 3 à 6, dans lequel
les informations (20) comprennent un clavier virtuel (12) de l'appareil médical et
le procédé comprend en outre l'étape consistant à :
actionner des mouvements du modèle 3D de l'appareil médical conformément à des entrées au clavier virtuel (12).

8. Procédé selon l'une quelconque des revendications précédentes, comprenant en outre les étapes consistant à :
capturer une image de l'appareil médical avec la caméra (4) ; et
déterminer le type de l'appareil médical en évaluant l'image capturée de l'appareil médical réel.

9. Procédé selon l'une quelconque des revendications précédentes, dans lequel
les informations (20) comprennent un modèle 3D d'un sous-ensemble de l'appareil médical ou d'un composant de l'appareil médical, et
le procédé comprend en outre l'étape consistant à :
tourner le modèle 3D du sous-ensemble ou du composant illustré par le dispositif d'illustration en touchant virtuellement le modèle 3D du sous-ensemble ou du composant via le dispositif d'illustration au moyen d'un ou de l'objet et en déplaçant l'objet.

10. Procédé selon l'une quelconque des revendications précédentes, dans lequel
les informations (2) comprennent au moins l'un d'entre un manuel d'entretien et un manuel utilisateur de l'appareil médical, et
le procédé comprend en outre l'étape consistant à :
sélectionner une zone dans le manuel d'entretien ou le manuel utilisateur en touchant virtuellement la zone au moyen d'un ou de l'objet et fournissant ainsi davantage de données sur cette zone.

11. Procédé selon l'une quelconque des revendications précédentes, dans lequel
les informations (20) comprennent un ou le clavier virtuel (12) de l'appareil médical, et
le procédé comprend en outre l'étape consistant à :
actionner des mouvements de l'appareil médical réel conformément à des entrées au clavier virtuel (12) via le dispositif de transmission et de réception (10).

12. Procédé selon l'une quelconque des revendications précédentes, dans lequel
les informations (2) comprennent un identifiant de l'appareil médical,
l'identifiant comprend au moins l'un d'entre un numéro de série de l'appareil médical et une version micrologicielle du logiciel,
l'identifiant est soit entré au dispositif intelligent soit détecté en évaluant des données transmises de l'appareil médical, et
le procédé comprend en outre l'étape consistant à :
récupérer les données conformément à l'identifiant d'une mémoire du dispositif intelligent (2) ou de l'appareil médical via le dispositif intelligent (2) au moyen du dispositif de transmission et de réception (10).

13. Système de réalité assisté par ordinateur (1) comprenant un appareil médical (3) comprenant un contrôleur (7) et des capteurs (8), un dispositif à distance (9), et un dispositif intelligent (2) ayant une caméra (4) et comprenant un dispositif de transmission et de réception (10) pour transmettre des données au et recevoir des données du dispositif intelligent (9) et de l'appareil médical (3), et un dispositif d'illustration pour illustrer une image, dans lequel le système de réalité assisté par ordinateur (1) est configuré pour exécuter un procédé selon l'une quelconque des revendications précédentes.

14. Système de réalité assisté par ordinateur selon la revendication 13, dans lequel
l'appareil médical est formé par une table chirurgicale (3).

15. Produit de programme informatique configuré pour mettre en œuvre un procédé selon l'une quelconque des revendications 1 à 12.
